# EUROPEAN PATENT SPECIFICATION

(11) **EP 1 927 346 B1**
(45) Date of publication and mention of the grant of the patent: **03.10.2018**
(21) Application number: 07021972.0
(22) Date of filing: 13.11.2007
(51) Int. Cl.: A61K 8/31, A61K 8/73, A61Q 5/06, A61K 8/92

(54) **Hair styling emulsion composition**
Haarstylingzusammensetzung in Emulsionsform
Composition de coiffage sous forme d'émulsion

(30) Priority: 16.11.2006 EP 06023811
(43) Date of publication of application: 04.06.2008
(73) Proprietor: Kao Germany GmbH, 64297 Darmstadt (DE)
(72) Inventor: Klutzny, Jutta, 64291 Darmstadt (DE); Bräutigam, Ina, 64297 Darmstadt (DE)

(56) References cited:
- EP-A- 0 948 960
- EP-A- 1 598 046
- WO-A-92/19219
- WO-A-02/074258
- US-A- 2 310 687
- US-A- 2 655 923
- US-A- 5 879 669
- DROVETSKAYA T V ET AL: "EFFECTS OF LOW-LEVEL HYDROPHOBIC SUBSTITUTION ON CONDITIONING PROPERTIES CATIONIC CELLULOSIC POLYMERS IN SHAMPOO SYSTEMS", JOURNAL OF COSMETIC SCIENCE, SOCIETY OF COSMETIC CHEMISTS, NEW YORK, NY, US, vol. 55, no. SUPPL, 1 January 2004 (2004-01-01), pages 195-205, XP009076338, ISSN: 1525-7886

## Description

The present invention is related to an emulsion composition for keratin fibres especially hair for conditioning, styling and restyling purposes.

Emulsions have been widely used for hair styling and/or restyling. Other than emulsions with sole conditioning effect on hair surface, styling polymers and certain inert filling substances are used. Examples to those filling substances are kaolin, kieselguhr etc. Compositions including such filling substances show volumizing and bodifying effects but hair styled with such compositions often feel unnatural, over conditioned and therefore not soft enough.

Among natural polymers starch derivatives have recently gained attention for their styling benefits. US 5879669 discloses aqueous hair fixing compositions containing a natural starch, emulsifier, an anionic film forming polymer and very low amounts of perfume oil.

The present inventors have surprisingly found out that a wax composition comprising natural starch, emulsifier and a film forming polymer in addition to wax have excellent hair styling and restyling benefits together with excellent hold, volumizing and bodifying effects. The hair feels natural upon touching and matt as starch takes away the shine effect of the wax like components.

Thus, the object of the present invention is an aqueous composition comprising at least one oil and/or wax at a concentration 10 to 40% by weight, calculated to total composition, at least one natural starch at a concentration 1 to 25% by weight, calculated to total composition, at least one emulsifier at a concentration of 0.05 to 10% by weight calculated to total composition and at least one film forming polymer selected from anionic, cationic and amphoteric polymers.

Another object of the present invention is use of an aqueous composition of the present invention for styling and restyling hair.

Still another subject of the present invention is process for styling hair wherein an aqueous composition of the present invention is applied onto wet and/or dry hair and hair is styled without rinsing the composition off.

With the term styling it is meant that the hair is freshly styled. With the term restyling it is meant that hair has already treated with an aqueous composition of the present invention and styled and after lapse of certain period of time a new style is given without using further composition of the present invention.

Thus, further object of the present invention is process for restyling hair wherein a new style is given to hair which is already styled with an aqueous composition of the present invention without applying any additional styling agent.

Compositions of the present invention comprise at least one oil and /or wax. In principal, any oil and wax allowed for cosmetic use is suitable for the compositions of the present invention.

Suitable non-limiting and preferred examples are petrolatum, ozokerit, carnauba wax, paraffin, lanolin wax, candelila wax, bees wax, microcrystalline wax and cocoglycerides.

Oils are those of synthetic and of natural ones. Natural oils are in principal any triglyceride suitable for cosmetic use. Non-limiting examples are avocado oil, coconut oil, palm oil, sesame oil, peanut oil, whale oil, sunflower oil, almond oil, peach kernel oil, wheat germ oil, macadamia nut oil, night primrose oil, jojoba oil, castor oil, or also olive oil, soya oil, and the derivatives thereof. Mineral oils such as paraffin oil and petrolatum are suitably contained within the scope of the present invention, It should as well be noted that compositions of the present invention can contain mixture of one or more natural oils and mineral oil.

Further, suitable synthetic oil components are in particular fatty alcohol fatty acid esters such as isopropyl myristate, palmitate, stearate and isostearate, oleyl oleate, isocetyl stearate, hexyl laurate, dibutyl adipate, dioctyl adipate, myristyl myristate, oleyl erucate, polyethylene glycol and polyglyceryl fatty acid esters, cetyl palmitate, etc.

Synthetic ones are those of silicone oils. Here again any silicone oil either volatile and/or non-volatile is suitable for the compositions of the present invention. Preferred silicone oils are non-volatile silicone oils known with their INCI name as dimethicone, dimethiconol, and phenyltrimethicone which is an arylated silicone oil. Volatile silicone oils such as cyclomethicones may be used in combination with non-volatile silicones and/or other wax and/or oils mentioned above. Commercially, they are available from various companies for example Dow Corning with the known DC series, Wacker Chemie and Toray silicones. All commercially available non volatile silicones are suitable in the compositions of the present invention. Examples to those are DC 200 series, DC 556, DC1401, DC 1403, DC 1501 and DC 1503. Concentration of wax and/or oils in the compositions of the present invention is between 10 and 40%, preferably 10 and 30% and most preferably 10 and 25% by weight calculated to total composition.

Compositions of the present invention comprise at least one natural starch at a concentration of 1 to 25%, preferably 1 to 20%, more preferably 2.5 to 20 and most preferably 5 to 15% by weight calculated to total composition. Within the meaning of the present invention any natural starch is suitable. Preferred are wheat, rice, potato, corn starches. Most preferred is rice starch.

Compositions of the present invention comprise at least one emulsifier. Suitable emulsifiers are of anionic, non-ionic, amphoteric and cationic surfactants or their mixtures. Preferred are cationic, non-ionic and anionic emulsifiers. Most preferred emulsifier is non-ionic emulsifier combined with anionic or cationic surfactants.

As a rule any cationic surfactant is suitable for the compositions of the present invention. Preferably at least one cationic surfactant is selected from the compounds with the general formula 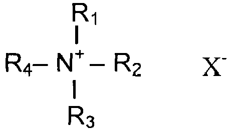 where R₁s a saturated or unsaturated, branched or non-branched alkyl chain with 8-22 C atoms or

R₅CO NH (CH₂)ₙ

where R₅ is saturated or unsaturated, branched or non-branched alkyl chain with 7-21 C atoms and n has typical value of 0 - 4 or

R₆CO O (CH₂)ₙ

where R₆ is saturated or unsaturated, branched or non-branched alkyl chain with 7-21 C atoms and n has typical value of 0 - 4, and
and R₂, R₃ and R₄ are independent from each other H or lower alkyl chain with 1 to 4 carbon atoms or ethoxy or propoxy group with number of ethoxy or propoxy groups varying in the range of 0 to 4, and X is chloride, bromide or methosulfate.

Suitable cationic surfactants and or conditioning agents are, for example, long-chain quaternary ammonium compounds which can be used alone or in admixture with one another, such as cetyl trimethyl ammonium chloride, myristoyl trimethyl ammonium chloride, behentrimonium chloride, trimethyl cetyl ammonium bromide, stearyl trimethyl ammonium chloride, dimethyl stearyl ammonium chloride, stearamidopropyldimethylamoonium chloride.

Suitable non-ionic surfactants are alkyl polyglucosides of the general formula

R₇-O-(R₈O)ₙO-Zₓ

wherein R₇ is an alkyl group with 8 to 18 carbon atoms, R₈ is an ethylene or propylene group, Z is a saccharide group with 5 to 6 carbon atoms, n is a number from 0 to 10 and x is a number between 1 and 5. Examples are decyl polyglucoside, cocoyl polyglucoside both are commercially available.

Further nonionic surfactant components are, for example, long-chain fatty acid mono- and dialkanolamides, such as coco fatty acid mono and di ethanolamide and myristic fatty acid mono and di ethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}".

Further nonionic surfactants as emulsifiers useful in the compositions according to invention are C₁₀-C₂₂-fatty alcohol ethoxylates. Suitable non-limiting examples are oleth-10, oleth-11, oleth-12, oleth-15, oleth-16, oleth-20, oleth-25, oleth-30, oleth-35, oleth-40, laureth-10, laureth-11, laureth-12, laureth-13, laureth-15, laureth-16, laureth-20, laureth-25, laureth-30, laureth-35, laureth-40, laureth-50, ceteth-10, ceteth-12, ceteth-14, ceteth-15, ceteth-16, ceteth-17, ceteth-20, ceteth-25, ceteth-30, ceteth-40, ceteth-45, cetoleth-10, cetoleth-12, cetoleth-14, cetoleth-15, cetoleth-16, cetoleth-17, cetoleth-20, cetoleth-25, cetoleth-30, cetoleth-40, cetoleth-45, ceteareth-10, ceteareth-12, ceteareth-14, ceteareth-15, ceteareth-16, ceteareth-18, ceteareth-20, ceteareth-22, ceteareth-25, ceteareth-30, ceteareth-40, ceteareth-45, ceteareth-50, isosteareth-10, isosteareth-12, isosteareth-15, isosteareth-20, isosteareth-22, isosteareth-25, isosteareth-50, steareth-10, steareth-11, steareth-14, steareth-15, steareth-16, steareth-20, steareth-25, steareth-30, steareth-40, steareth-50, steareth-80 and steareth-100. Additional examples of similar compounds can be found in the cosmetic ingredient dictionaries and cosmetic textbooks.

Further non-ionic emulsifiers within the meaning of the present invention are polyalkyleneglycol ether of fatty acid glyceride or partial glyceride with at least 30 polyalkylene units are with 30 to 1000, preferably 30 to 500, more preferably 30 to 200 and most preferably 40 to 100 polyethyleneglycol units. Examples to those are PEG-30 hydrogenated castor oil, PEG-35 hydrogenated castor oil, PEG-40 hydrogenated castor oil, PEG-45 hydrogenated castor oil, PEG-50 hydrogenated castor oil, PEG-55 hydrogenated castor oil, PEG-60 hydrogenated castor oil, PEG-65 hydrogenated castor oil, PEG-80 hydrogenated castor oil, PEG-100 hydrogenated castor oil, PEG-200 hydrogenated castor oil, PEG-35 castor oil, PEG-50 castor oil, PEG-55 castor oil, PEG-60 castor oil, PEG-80 castor oil, PEG-100 castor oil, PEG-200 castor oil. Additional examples of similar compounds can be found in the cosmetic ingredient dictionaries and cosmetic textbooks.

Further suitable non-ionic emulsifiers are monoglycerides such as glyceryl stearate, glyceryl palmitate, glyceryl myristate, glyceryl behenate.

Among the non-ionic surfactants mentioned above fatty alcohol ethoxylates are the most preferred ones.

As further surfactant component as emulsifier, the compositions according to the invention can also contain amphoteric or zwitterionic surfactants. Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate.

Further emulsifiers suitable within the meaning of the present invention are anionic surfactants of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono-, di- or tri alkyl phosphates constituting mild, skin-compatible detergents.

Additional anionic surfactants useful are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₉-(C₂H₄O)ₙ-O-CH₂COOX,

wherein R₉ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyt asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

Concentration of emulsifier in the compositions according to present invention is in the range of 0.05 to 10%, preferably 0.1 to 7.5% and more preferably 0.25 to 5% by weight calculated to total composition.

The compositions of the present invention preferably comprise at least one film forming polymer selected from the anionic, cationic and/or amphoteric ones.

As amphoteric polymers which can be used alone or in mixture with at least one additional nonionic polymer, reference is here made in particular to copolymers of N-octyl acrylamide, (meth)acrylic acid and tert.-butyl aminoethyl methacrylate of the type "Amphomer®"; copolymers from methacryloyl ethyl betaine and alkyl methacrylates of the type "Yukaformer®", e.g.. the butyl methacrylate copolymer "Yukaformer® Am75"; copolymers from monomers containing carboxyl groups and sulfonic groups, e.g.. (meth)acrylic acid and itaconic acid, with monomers such as mono- or dialkyl aminoalkyl (meth)acrylates or mono- or dialkyl aminoalkyl (meth)-acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from USA 3,927,199.

Suitable anionic polymers alone or in combination with non-ionic polymers are vinyl - alkyl ether, in particular methyl vinyl ether/maleic acid copolymers, obtained by hydrolysis of vinyl ether/maleic anhydride copolymers, distributed under the trade name "Gantrez® AN or ES". These polymers may also be partly esterified, as for example, "Gantrez® ES 225" or "ES 435", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn®"; sodium acrylate/vinyl alcohol copolymers of the type "Hydagen® F", sodium polystyrene sulfonate, e.g.. "Flexan® 130"; ethyl acrylate/acrylic acid/N-tert.-butyl acrylamide copolymers of the type "Ultrahold®"; vinyl pyrrolidone/vinyl acetate/itaconic acid copolymers, acrylic acid/acrylamide copolymers or the sodium salts thereof of the type "Reten®"; etc.

Further suitable anionic polymers are Acrylate copolymers available under trade name Salcare SC 81, PEG/PPG 25/25 dimethicone / acrylate copolymer available under trade name Luviglex Silk from BASF, Acrylates/t-butylacrylamide copolymer available under trade name Ultrahold Strong, Advantage LC-E which is vinylcaprolactam/PVP/dimethylaminoethylmethacrylate copolymer and VA/crotonates copolymer available under trade name Luviset CA 66.

Composition of the present invention can comprise cationic polymers alone or in combination with non-ionic polymer. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore. chitosan and chitin can also be included in the compositions as cationic natural polymers.

Furthermore, those cationic polymers known with their CTFA category name Polyquaternium may as well be added into the compositions of the present invention. Typical examples of those are Polyquaternium 6, Polyquaternium 7, Polyquaternium 10, Polyquaternium 11, Polyquaternium 16, Polyquaternium 22 and Polyquaternium 28, Polyquaternium 30, Polyquaternium 37, Polyquaternium 36, Polyquaternium 46.

As well those polymers known with their CTFA category name Quaternium can as well be suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

In this context, reference is also made to the cationic polymers disclosed in DE 25 21 960, 28 11 010, 30 44 738 and 32 17 059, as well as to the products described in EP-A 337 354 on pages 3 to 7, It is also possible to use mixtures of various cationic polymers.

The cationic polymers also include the quaternized products of graft polymers from organopolysiloxanes and polyethyl oxazolines described in EP-A 524 612 and EP-A 640 643. Among these especially preferred is the compound know with the INCI name Polysilicone-9.

Concentration of polymers of anionic, cationic and/or amphoteric or zwitterionic character is in the range of 1 - 20%, preferably 2 - 15% and more preferably 3 - 12% and most preferably 4 - 10% by weight, calculated to the total composition.

The compositions of the present invention can contain one or more organic solvents within the scope of the invention, Suitable ones are ethanol, propanol, isopropanol, isopentane, n-pentane, n.hexane, dimethoxymethane, benzyl alcohol, benzyloxyethanol, alkylene carbonates such as ethylene carbonate and propylene carbonate, phenoxyethanol, butanol, isobutanol, cyclohexane, cyclohexanol, ethylenecarbonate, ethyleneglycol monoethylether, ethylene glycol monobutyl ether, ethylene glycol monophenyl ether, 1-phenylethylalcohol, 2-phenylethylalcohol, o-methoxyphenol. The most preferred organic solvents are ethanol, isopropanol and propanol without limiting the scope. Concentration of solvents is in the range of 1 to 25% and preferably 2.5 to 20%, more preferably 5 to 15% and most preferably 5 to 10% by weight calculated to total composition.

The composition of the present invention can comprise polyols at a concentration of 0.5 to 15%, preferably 1 to 10%, more preferably 2 to 5% by weight calculated to the total concentration. The most preferred ones are glycerine, propylene glycols, butylene glycol and hexylene glycol.

Cationic silicones such as the one know with INCI name as amodimethicone can as well be contained in the compositions of the present invention. Commercially it is available under the trade name DC 949 in emulsified form in mixture with a nonionic surfactant and a cationic surfactant.

The compositions according to the invention may also comprise further agents, such as proteins, for example bamboo protein, and protein hydrolyzates and polypeptides, e.g. keratin hydrolyzates, collagen hydrolyzates of the type "Nutrilan®" or elastin hydrolyzates, as well as, in particular vegetable, optionally cationized protein hydrolyzates, for example "Gluadin®.

Additional natural plant extracts can as well form part of the compositions of the present invention. Those are incorporated usually in an amount of about 0.01 % to about 5 %, preferably 0.05 % to 3.5 %, in particular 0.1 % to 2 % by weight, calculated as dry residue thereof to the total composition. Suitable aqueous (e.g. steam-distilled) alcoholic or hydro-alcoholic plant extracts known **per se** are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, coconut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapon®" products, "Herbasol^{R}", "Sedaplant^{R}" and "Hexaplant^{R}". Extracts and the preparation thereof are also described in "Hagers Handbuch der pharmazeutischen Praxis". 4^{th} Ed..

Compositions of the present invention may contain UV filters either for stabilization of the product colour and/or for protection of hair from environmental influences such as loss of elasticity, loss of hair colour (bleaching effect of sun light). Suitable UV-absorbing substance are 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2,4-dihydroxybenzophenone, 2,2',4,4'-tetrahydroxy- benzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4,4'-dimethoxy-5,5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher.

The suitable amount of the UV-absorber ranges from about 0.01 % to 1% by weight, calculated to the total composition. Attention should be paid to the stability and solubility especially when using UV filter as salts, e.g. anionic UV filter salts.

Compositions of the present invention may further comprise polyethyleneglycols Suitable non- limiting examples are PEG-14, PEG-20, PEG-23, PEG-25, PEG-90, PEG-115, PEG-160, PEG-14M, PEG-20M, PEG-23M, PEG-25M, PEG-90M, PEG-115M, PEG-160M, etc.

In a further embodiment of the present invention, the compositions comprise at least one direct dye for colouring hair. Suitable direct dyes are cationic, anionic, neutral dyes and their mixtures as available commercially from various suppliers and used mainly in semipermanent hair coloration.

Cationic dyes are the most preferred ones for the purpose of dyeing hair. Non-limiting examples are Basic Blue 6, Basic Blue 7, Basic Blue 9, Basic Blue 26, Basic Blue 41, Basic Blue 99, Basic Brown 4, Basic Brown 16, Basic Brown 17, Basic Orange 31, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

Further suitable direct dyes are anionic dye. Suitable nonlimiting examples are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Further suitable dyes for colouring hair within the meaning of the present invention are those of neutral nitro dyes. Suitable non-limiting examples are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No. 10, HC Yellow No.11, HC Yellow No. 12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs may also be used as hair colorant within the meaning of the present invention for example henna (red or black), alkanna root, laccaic acid, indigo, logwood powder, madder root and rhubarb powder, etc.

It should be noted that the above dyestuffs are also suitable for use in mixture. When using direct dyes of various categories, their compatibility must be checked.

Concentration of direct dyes in the compositions of the present invention is within the range of 0.001 to 5%, preferably 0.01 to 3% and more preferably 0.05 to 2%, and most preferably 0.1 to 1% by weight calculated to total composition.

Concentration of the high molecular weight polyethyleneglycol, one or mixture of more than one, is in the range of 0.05% to 2.5%, preferably 0.1% to 1.5% and most preferably between 0.1 to 1.0% by weight calculated to total composition.

pH of the compositions vary between 3 to 9, preferably 4 to 8 and more preferably 5 to 8, and most preferably 6 to 7, measured 25°C.

Furthermore compositions of the present invention can comprise all substances customarily found in such preparations. Examples of such substances are complexing agents, preservatives, fragrances, etc.

The following examples are to illustrate the invention but not to limit.

### Example 1 (not within the scope of the claims)

| | % by weight |
|---|---|
| Petrolatum | 10.00 |
| Ozokerite | 10.00 |
| Oryza Sativa (Rice) Starch | 5.00 |
| Ceteareth-20 | 3.00 |
| Benzophenone-4 | 0.05 |
| Fragrance, preservative | q.s. |
| Sodium hydroxide/Lactic acid | q.s. to pH 6.5 |
| Dyestuff | q.s. to color product |
| Water | q.s to 100 % |

The above composition was prepared as follows: Waxes are melted and mixed with starch at approximately 80°C. In another vessel water phase was heated to the temperature of wax phase and mixed with wax phase which subsequently homogenised. Afterwards the mixture was cooled down to 65°C and pH was adjusted and fragrance was added. The mixture so obtained was cooled down to room temperature without mixing.

The above composition is applied onto dry hair and. It was observed that hair can be styled easily and shine was enhanced significantly.

The above composition can be prepared with other natural starches such as wheat, potato or corn or their mixture.

### Example 2

| | % by weight |
|---|---|
| Prunus Amygdalus Dulcis (Sweet Almond)Oil | 5.00 |
| Mineraloil | 10.00 |
| Oryza Sativa (Rice) Starch | 5.00 |
| Laureth-23 | 3.00 |
| Acrylates/Octylacrylamide Copolymer (Amphomer HC) | 2.00 |
| Butylene Glycole | 1.50 |
| Propylenglycol | 2.00 |
| Sodium hydroxide | q.s. to pH 8.0 |
| Fragrance, preservative | q.s. |
| Water | q.s to 100 % |

The above composition was prepared in a similar way as to Example 1 except that Amphomer HC was neutralised 100% in aqueous phase before combining with wax phase.

## Claims

1. An aqueous composition for keratin fibres especially for human hair **characterised in that** it comprises at least one oil and/or wax at a concentration 10 to 40% by weight calculated to total composition, at least one natural starch at a concentration 1 to 25% by weight calculated to total composition, at least one emulsifier at a concentration of 0.05 to 10% by weight calculated to total composition and at least one film forming polymer selected from anionic, cationic and amphoteric polymers.

2. Composition according to claim 1 **characterised in that** wax is selected from petrolatum, ozokerit, carnauba wax, paraffin, lanolin wax, candelila wax, bees wax, microcrystalline wax and cocoglycerides.

3. Composition according to claims 1 and 2 **characterised in that** oil is selected from natural and synthetic oil.

4. Composition according to any of the preceding claims **characterised in that** natural starch is selected from wheat, rice, potato, corn starches, and mixtures thereof.

5. Composition according to claim 4 **characterised in that** natural starch is rice starch.

6. Composition according to any of the preceding claims **characterised in that** at least one emulsifier is selected from anionic, cationic, non-ionic and amphoteric surfactants.

7. Composition according to claim 6 **characterised in that** emulsifier is mixture of at least one non-ionic surfactant and anionic or cationic surfactants.

8. Composition according to any of the preceding claims **characterised in that** it comprises emulsifiers at a concentration of 0.1 to 7.5% by weight calculated to total composition.

9. Composition according to any of the preceding claims **characterised in that** it comprises one or more organic solvents.

10. Composition according to any of the preceding claims **characterised in that** it comprises polyol.

11. Composition according to any of the preceding claims **characterised in that** it comprises at least one UV filter.

12. Composition according to any of the preceding claims **characterised in that** it comprises at least one direct dye.

13. Use of an aqueous composition according to claims 1 to 12 for styling and restyling hair.

14. Process for styling hair wherein a composition according to claims 1 to 12 is applied onto wet and/or dry hair and hair is styled without rinsing the composition off.

15. Process for restyling hair wherein a new style is given to hair which already styled with a composition according to claims 1 to 12 without applying any additional styling agent.

## Patentansprüche

1. Wässrige Zusammensetzung für Keratinfasern, insbesondere für menschliche Haare, **dadurch gekennzeichnet, dass** sie mindestens ein Öl und/oder Wachs in einer Konzentration von 10 bis 40 Gewichts-%, bezogen auf die Gesamtzusammensetzung, mindestens eine natürliche Stärke in einer Konzentration von 1 bis 25 Gewichts-%, bezogen auf die Gesamtzusammensetzung, mindestens einen Emulgator in einer Konzentration von 0,05 bis 10 Gewichts-%, bezogen auf die Gesamtzusammensetzung, und mindestens ein filmbildendes Polymer aufweist, ausgewählt aus anionischen, kationischen und amphoteren Polymeren.

2. Zusammensetzung nach Anspruch 1, **dadurch gekennzeichnet, dass** das Wachs aus Rohvaseline, Ozokerit, Karnaubawachs, Paraffin, Lanolinwachs, Kandelillawachs, Bienenwachs, mikrokristallinem Wachs und Kokosglyceriden ausgewählt ist.

3. Zusammensetzung nach Anspruch 1 und 2, **dadurch gekennzeichnet, dass** das Öl aus natürlichem und synthetischem Öl ausgewählt ist.

4. Zusammensetzung nach einem der vorhergehenden Ansprüche, **dadurch gekennzeichnet, dass** die natürliche Stärke aus Weizen-, Reis-, Kartoffel-, Maisstärken und Gemischen davon ausgewählt ist.

5. Zusammensetzung nach Anspruch 4, **dadurch gekennzeichnet, dass** die natürliche Stärke Reisstärke ist.

6. Zusammensetzung nach einem der vorhergehenden Ansprüche, **dadurch gekennzeichnet, dass** mindestens ein Emulgator aus anionischen, kationischen, nichtionischen und amphoteren Tensiden ausgewählt ist.

7. Zusammensetzung nach Anspruch 6, **dadurch gekennzeichnet, dass** der Emulgator ein Gemisch aus mindestens einem nichtionischen Tensid und anionischen oder kationischen Tensiden ist.

8. Zusammensetzung nach einem der vorhergehenden Ansprüche, **dadurch gekennzeichnet, dass** sie Emulgatoren in einer Konzentration von 0,1 bis 7,5 Gewichts-% aufweist, bezogen auf die Gesamtzusammensetzung.

9. Zusammensetzung nach einem der vorhergehenden Ansprüche, **dadurch gekennzeichnet, dass** sie ein oder mehrere organische Lösungsmittel aufweist.

10. Zusammensetzung nach einem der vorhergehenden Ansprüche, **dadurch gekennzeichnet, dass** sie Polyol aufweist.

11. Zusammensetzung nach einem der vorhergehenden Ansprüche, **dadurch gekennzeichnet, dass** sie mindestens einen UV-Filter aufweist.

12. Zusammensetzung nach einem der vorhergehenden Ansprüche, **dadurch gekennzeichnet, dass** sie mindestens einen Direktfarbstoff aufweist.

13. Verwendung einer wässrigen Zusammensetzung nach Anspruch 1 bis 12 zum Styling und Re-Styling von Haaren.

14. Verfahren zum Styling von Haaren, wobei eine Zusammensetzung nach Anspruch 1 bis 12 auf feuchte und/oder trockene Haare aufgebracht wird und die Haare gestylt werden, ohne die Zusammensetzung auszuspülen.

15. Verfahren zum Re-Styling von Haaren, wobei Haare, welche bereits mit einer Zusammensetzung nach Anspruch 1 bis 12 gestylt wurden, ohne Aufbringen eines zusätzlichen Styling-Mittels neu gestylt werden.

## Revendications

1. Composition aqueuse pour fibres de kératine, en particulier, pour cheveux humains, **caractérisée en ce qu'**elle comprend au moins une huile et/ou une cire à une concentration de 5 à 40 % en poids, calculée par rapport à la composition totale, au moins un amidon naturel à une concentration de 1 à 25 % en poids, calculée par rapport à la composition totale, au moins un émulsifiant à une concentration de 0,05 à 10 % en poids, calculée par rapport à la composition totale, et au moins un polymère filmogène choisi parmi des polymères anioniques, cationiques et amphotères.

2. Composition selon la revendication 1, **caractérisée en ce que** la cire est choisie parmi le pétrolatum, l'ozokérite, la cire de carnauba, la paraffine, la cire de lanoline, la cire de candelilla, la cire d'abeille, la cire microcristalline et des glycérides de coco.

3. Composition selon les revendications 1 et 2, **caractérisée en ce que** l'huile est choisie parmi une huile naturelle et une huile synthétique.

4. Composition selon l'une quelconque des revendications précédentes, **caractérisée en ce que** l'amidon naturel est choisi parmi les amidons de blé, de riz, de pomme de terre, de maïs et des mélanges de ceux-ci.

5. Composition selon la revendication 4, **caractérisée en ce que** l'amidon naturel est de l'amidon de riz.

6. Composition selon l'une quelconque des revendications précédentes, **caractérisée** en qu'au moins un émulsifiant est choisi parmi des tensioactifs anioniques, cationiques, non ioniques et amphotères.

7. Composition selon la revendication 6, **caractérisée en ce que** l'émulsifiant est un mélange d'au moins un tensioactif non ionique et de tensioactifs anioniques ou cationiques.

8. Composition selon l'une quelconque des revendications précédentes, **caractérisée en ce qu'**elle comprend des émulsifiants à une concentration de 0,1 à 7,5% en poids, calculée par rapport à la composition totale.

9. Composition selon l'une quelconque des revendications précédentes, **caractérisée en ce qu'**elle comprend un ou plusieurs solvants organiques.

10. Composition selon l'une quelconque des revendications précédentes, **caractérisée en ce qu'**elle comprend un polyol.

11. Composition selon l'une quelconque des revendications précédentes, **caractérisée en ce qu'**elle comprend au moins un filtre UV.

12. Composition selon l'une quelconque des revendications précédentes, **caractérisée en ce qu'**elle comprend au moins un colorant direct.

13. Utilisation d'une composition selon les revendications 1 à 12 pour la mise en forme et la remise en forme des cheveux.

14. Procédé de mise en forme des cheveux, dans lequel une composition selon les revendications 1 à 12 est appliquée sur les cheveux humides et/ou secs et les cheveux sont mis en forme sans enlever la composition par rinçage.

15. Procédé de remise en forme des cheveux, dans lequel un nouveau style est donné aux cheveux qui ont déjà été mis en forme avec une composition selon les revendications 1 à 12 sans appliquer un quelconque agent supplémentaire de mise en forme.
